# EUROPEAN PATENT SPECIFICATION

(11) **EP 4 020 488 B1**
(45) Date of publication and mention of the grant of the patent: **04.11.2026**
(21) Application number: 22157359.5
(22) Date of filing: 18.03.2020
(51) Int. Cl.: G16H 10/60, G16H 50/20, G06N 3/04, G06N 3/08, G06N 3/082, G06N 3/0499, G06N 3/09, G06N 3/091, G16H 40/63, G16H 40/67

(54) **TECHNIQUE FOR EFFICIENT RETRIEVAL OF PERSONALITY DATA**
VERFAHREN ZUM EFFIZIENTEN ABRUFEN VON PERSÖNLICHKEITSDATEN
TECHNIQUE DE RÉCUPÉRATION EFFICACE DE DONNÉES DE PERSONNALITÉ

(30) Priority: 19.03.2019 EP 19163909
(43) Date of publication of application: 29.06.2022
(62) Document number(s) of the earlier application(s) in accordance with Art. 76 EPC: 20713243.2 / 3 942 561
(73) Proprietor: 2HFutura SA, N8 Panama City (PA)
(72) Inventor: Giersch, Daniel, 98000 Monaco (MC)
(74) Representative: Laqua, Bernd Christian Kurt

(56) References cited:
- WO-A1-2019/000326
- US-A1- 2008 033 826
- US-A1- 2014 309 790
- ILMINI W M K S ET AL: "Computational personality traits assessment: A review", 2017 IEEE INTERNATIONAL CONFERENCE ON INDUSTRIAL AND INFORMATION SYSTEMS (ICIIS), IEEE, 15 December 2017 (2017-12-15), pages 1 - 6, XP033324699, ISBN: 978-1-5386-1674-1, [retrieved on 20180221], DOI: 10.1109/ICIINFS.2017.8300416

## Description

### Technical Field

The present disclosure generally relates to the field of data retrieval. In particular, a technique for enabling efficient retrieval of a digital representation of personality data of a user by a client device from a server is presented. The technique may be embodied in methods, computer programs, apparatuses and systems.

### Background

Personality tests have been used for decades to assess people's personality characteristics and are typically performed based on personality survey data obtained from a person to be tested, wherein the survey data is evaluated by a professional, such as a psychologist, to conclude on the person's personality. The so called "OCEAN" model is a widely accepted taxonomy for personality traits, also known as the "Big Five" personality traits, and includes openness, conscientiousness, extraversion, agreeableness and neuroticism as personality dimensions. Widely known personality tests using the OCEAN model include tests based on the so called International Personality Item Pool (IPIP), the HEXACO-60 inventory and the Big-Five-Inventory-10 (BFI-10), for example, which comprise sets of questions for testing a person on each of the five personality dimensions. As conventional personality tests generally require a review by a human professional, such as a psychologist, to obtain a qualified assessment of a person's personality traits, however, it is difficult to integrate carrying out personality tests and their results into processes performed on technical systems, although such integration could be beneficial because it would allow adapting processes to better fit a user's personality and, therefore, to improve user experience, such as by providing user-adapted services to the user, for example.

Patent application US 2014/0309790 A1 discloses systems that provide an individual or group of individuals with an intuitive and comfortable vehicular environment. According to an embodiment, a virtual personality of a user may be retrieved and presented to the user. One or more features of the vehicle may be altered to change a mood associated with the virtual personality. For example, a personality module may communicate with a vehicle control system to change an internal lighting, an infotainment setting, a temperature, an oxygen level, an air composition, a comfort setting, a seat position, a transmission setting, and a navigation output.

Non-patent document W. M. K. S. Ilmini et al: "Computational Personality Traits Assessment: A Review", 2017 IEEE International Conference on Industrial and Information Systems (ICIIS), IEEE, 15 December 2017, pages 1-6, XP033324699, discloses methods and theories involved in psychological traits assessment and evolution of computational psychological traits assessment with different machine learning algorithms and different feature sets.

### Summary

Accordingly, there is a need for a technical implementation which makes the integration of personality tests and their results into processes performed on technical systems practically feasible.

The invention is defined by the appended independent claims. Preferred embodiments are recited in the dependent claims.

According to a first aspect, a method including a retrieval of a digital representation of personality data of a user by a client device from a server according to independent claim 1, to which the reader shall now refer, is provided.

By storing a trained neural network on the server and applying it for computing personality data of a user, retrieval of a digital representation of the personality data of the user may be made automatable (as conventional human reviews may no longer be needed) and, as such, an integration of the retrieval and use of users' personality data into (e.g., automated) processes performed on technical systems may become feasible. In particular, the neural network may be seen as an efficient functional data structure which enables computing the requested personality data in a single computational run, i.e., by inputting the input obtained from the user at the input nodes of the neural network and reading off the resulting output values representative of the personality data from the output nodes of the neural network. As such, the neural network may enable an efficient provision of personality data in the form of a digital representation to the client device, where it may be used to provide a service adapted to the particular personality of the user, to thereby improve user experience on the side of the client device. Due to the efficient provision of data, the integration of retrieval and use of personality data may especially become practical as the digital representation of the personality data may be provided to the client device without significant delay and may be processed at the client device instantly. A technical implementation may therefore be achieved which generally makes integrating the retrieval and use of personality data into processes performed on technical systems practically feasible.

The personality data of the user is indicative of psychological characteristics and/or preferences of the user and, as such, the personality data may generally include psychological data as well as medical data (e.g., data indicating a tendency to curiosity, anxiety, depression, etc.), including classical personality data which may be based on the personality dimensions of openness, conscientiousness, extraversion, agreeableness and neuroticism (known as the Big Five, as described above), for example. The digital representation of the personality data of the user may comprise a digital representation of the mentioned characteristics, such as a digital representation of at least one of the personality dimensions of openness, conscientiousness, extraversion, agreeableness and neuroticism, as computed by the neural network for the user, for example.

The client device may be configured to process the digital representation of the personality data for the purpose of enabling the provision of a user-adapted service to the user. In one variant, the client device may itself be configurable based on the digital representation of the personality data. An exemplary device which may be configurable by the digital representation of the personality data may be a vehicle, for example. The vehicle may in this case be the client device. The vehicle may process the received digital representation of the personality data of the user (e.g., a driver of the vehicle) and configure itself (e.g., including subcomponents thereof) so as to adapt the vehicle's driving configuration to the personality of the driver and to thereby provide a driving service that is specifically adapted to the personality of the user. If the personality data indicates that the driver tends to be risk-averse or anxious, for example, the vehicle's driving configuration may be configured to be more safety-oriented, whereas for drivers that tend to have a more risk-seeking personality, the vehicle's driving configuration may be configured to be more sporty. To this end, among other settings, the gas and brake reaction behavior of the vehicle may be adapted accordingly. Subcomponents of the vehicle providing vehicle-related services may be configured based on the personality data as well, such as a sound system of the vehicle including its sound and volume settings to better comply with the user's personality, for example.

In another variant, the client device may configure at least one other device based on the digital representation of the personality data, e.g., when it is the at least one other device that provides the service to the user. In such a variant, the client device may be a mobile terminal (e.g., a smartphone), for example, which may interface (e.g., using Bluetooth) with the vehicle (i.e., in this case, the vehicle corresponds to the at least one other device) and, upon receipt of the digital representation of the personality data from the server, the mobile terminal may configure the vehicle via the interface. It may thus be said that the digital representation of the personality data of the user may be processed at the client device to configure at least one device providing a service to the user. Configuring the at least one device may comprise configuring at least one setting of the at least one device and/or configuring at least one setting of a service provided by the at least one device. It will be understood that the vehicle is merely an example of a device which may be configurable based on personality data and that the client device and/or the at least one other device may correspond to other types of devices as well.

The method performed by the server further comprises receiving feedback characterizing the user, updating the neural network based on the feedback, and sending, to the client device, a digital representation of second personality data of the user, wherein the second personality data of the user is computed using the updated neural network. The digital representation of the second personality data of the user is processed at the client device to refine a configuration of the at least one device providing the service to the user (e.g., one of the configurations of the vehicle mentioned above). The feedback may be gathered at the client device and/or at the at least one device providing the service to the user and is indicative of the personality of the user. The feedback may include behavioral data reflecting behavior of the user monitored at the at least one device when using the service provided by the at least one device, for example, wherein, in one variant, the behavioral data may be monitored using (e.g., sensor based) measurements performed by the at least one device providing the service to the user. In the vehicle example, the behavior of the user being monitored may be the driving behavior of the user and the driving behavior may be measured by sensors at the vehicle, for example. For measuring the driving behavior, the sensors may sense the user's brake reaction and intensity, for example, and, since such measurements may be indicative of a user's personality (e.g., aggressiveness in driving), this information may be sent as feedback to the server in order to update the neural network and thereby refine the neural network's capability to compute personality data of users.

Updating the neural network includes training the neural network based on the feedback received from the client device, wherein, if the feedback represents a new input value which has not yet been input to the neural network, a new input node may be added to the neural network and the new input value may be assigned to the new input node when training the neural network. This makes the power of the neural network as an efficient functional data structure employed in the technical implementation presented herein especially apparent: the neural network represents an efficiently updatable data structure which may be updated based on arbitrary feedback on the user's personality received from the client device to refine its capability to compute personality data. The information conveyed by the feedback can be directly integrated into the neural network and may, once trained, immediately be reflected in subsequent requests sent to the server requesting digital representations of personality data. Conventional personality assessment techniques are rather fixed and may not support such updatability at all.

The digital representation of the personality of the user sent from the server to the client device may correspond to a digital representation of the personality of the user which was previously computed by the server upon a previous request for computing the personality of the user (e.g., upon performing a personality test by answering a set of questions by the user). The personality data of the user may thus be computed prior to receiving the request from the client device, wherein the request may include an access code previously provided by the server to the user upon computing the personality data of the user, wherein the access code allows the user to access the digital representation of the personality data of the user from different client devices. Such implementation may save computational resources at the server since the digital representation of the personality of the user may not have to be computed anew each time the digital representation of the personality data for that particular user is requested from a client device, but may be returned on the basis of the pre-computed personality data. The user, in turn, may use the access code to access the digital representation of personality data from a plurality of different client devices, such as from different vehicles the user may drive, e.g., a car and a motorcycle, or other types of devices.

The input obtained from the user may correspond to digital scores reflecting answers to questions regarding at least one of personality, goals and motivations of the user (as obtained in a question answer scheme in the manner of a personality test, for example), wherein each digital score may be used as input to a separate input node of the neural network when computing the personality data of the user using the neural network. The digital scores may correspond to a five level Likert scale having values from 1 to 5, for example. The neural network may correspond to a deep neural network having at least two hidden layers between the input layer comprising the input nodes and the output layer comprising the output nodes of the neural network. The questions relating to personality may correspond to questions of the conventional IPIP, HEXACO-60 and/or BFI-10 pools, for example, but it will be understood that other questions regarding the personality of the user may be used as well, including questions on psychological characteristics and/or preferences of the user. Questions specifically relating to goals and motivations of the user may define additional dimensions (e.g., in addition to the Big Five) that may increase the accuracy of the computed personality data over the conventional IPIP, HEXACO-60 and BFI-10 techniques. The network may be trained based on data collected in a basic survey conducted with a plurality of test persons (e.g., 1000 or more), wherein the basic survey may be carried out using the questions mentioned above.

In order to reduce the computational complexity when computing the personality data of the user, the neural network may be designed to have a specific network structure. In view of the context of the above questions, the structure of the neural network may generally be designed such that the number of input nodes is reduced as compared to the number of input nodes available when all of the above questions were used. The questions may thus correspond to questions selected from a set of questions representative of an optimally achievable result of computing personality data of a user (i.e., if all questions in the set of questions were answered by the user), wherein the selected questions may correspond to questions of the set of questions which are determined to be most influential with respect to the optimally achievable result. As each answer to a question may be input to a separate input node of the neural network, as described above, selecting a subset of the set of questions may reduce the number of input nodes when computing the personality data to thereby reduce the computational complexity. Due to the fact that questions which are most influential with respect to the achievable result are selected, the accuracy of the result output by the neural network may approximately be maintained.

In fact, tests have shown that the number of questions may be reduced drastically without significantly sacrificing result accuracy. Taking, as the set of questions being representative of an optimally achievable result of computing personality data, a set of questions which comprises the standard IPIP, HEXACO-60 and BFI-10 questions (summing up to a total number of 370 questions), optionally supplemented by further questions regarding goals and motivations of the user (resulting in a number of more than 370 questions in total), tests have shown that, when only the 30 most influential questions are used, approximately 90% of the accuracy of the optimally achievable result can be achieved. As such, the number of the selected questions may be less than 10% (preferably less than 5%) of the number of questions included in the set of questions being representative of the optimally achievable result. As, in this case, the number of input nodes of the neural network may be reduced extensively, computational resources may be saved significantly and personality data may be computed more efficiently.

In order to determine the questions of the set of questions which are most influential with respect to the optimally achievable result, in one variant, the questions may be selected from the set of questions based on correlating results achievable by each single question of the set of questions with the optimally achievable result and selecting questions from the set of questions which have the highest correlation with the optimally achievable result. A fixed subset of the set of questions being representative of the optimally achievable result may thus be determined, which may then be used to train the neural network with a reduced number of input nodes, as described above.

As said, the optimally achievable result may correspond to a result which is achieved if all questions in the set of questions were answered by the user, such as the set of questions comprising the standard IPIP, HEXACO-60 and BFI-10 questions, optionally supplemented by further questions regarding goals and motivations of the user, as described above. While, in one variant, the standard IPIP scores (as obtained by answering all questions in the standard IPIP test), the standard HEXACO-60 scores (as obtained by answering all questions in the standard HEXACO-60 test) and the standard BFI-10 scores (as obtained by answering all questions in the standard BFI-10 test) may be taken individually as reference for the optimally achievable result, in another variant, an improvement may be achieved by calculating a combined score of these individual scores as reference for the optimally achievable result, wherein the combined score may be calculated as a (e.g., weighted) average of the individual scores, for example. The combined score may also be denoted as a "superscore" representative of the "truth" derivable from the individual scores, generally improving the meaningfulness of the determined score and representing an improved reference for the optimally achievable result.

In another variant, the questions may be selected iteratively from the set of questions, wherein, in each iteration, a next question may be selected depending on an answer of the user to a previous question, and wherein, in each iteration, the next question may be selected as a question of the set of questions which is determined to be most influential on an achievable result for computing personality data of the user. This may be seen as an adaptive selection of the questions, wherein questions are determined user specifically in a stepwise manner taking into account answers to previous questions of the user. In one particular variant, the neural network may comprise a plurality of output nodes representative of a probability curve of a result of the personality data of the user, wherein determining the most influential question of the set of questions as the next question of the respective iteration may include determining, for each input node of the neural network, a degree according to which a change in the digital score which is input to the respective input node of the neural network changes the probability curve. The question associated with an input node for which the degree of change in the probability curve is determined to be highest may be selected as the most influential question for the respective iteration.

To further reduce computational complexity, the above iterative and adaptive selection may be performed under at least one constraint, such as at least one of a maximum number of questions to be selected, a minimum result accuracy to be achieved (the result accuracy may increase with each answered question per iteration and, when the desired minimal result accuracy is reached, the computation may be stopped), and a maximum available time (the test may be stopped upon lapse of the maximum available time, or each question may be associated with an estimated time to be answered by the user and the number of questions to be selected may be determined based on the estimated times). These constraints may be configurable for each computation of personality data separately.

According to a second aspect, a method including a retrieval of a digital representation of personality data of a user by a client device from a server according to independent claim 10, to which the reader shall now refer, is provided.

The method according to the second aspect defines a method from the perspective of a client device which may be complementary to the method performed by the server according to the first aspect. The server and the client device of the second aspect may correspond to the server and the client device described above in relation to the first aspect. As such, those aspects described with regard to the method of the first aspect which are applicable to the method of the second aspect may be comprised by the method of the second aspect as well, and vice versa. Unnecessary repetitions are thus omitted in the following.

As in the method of the first aspect, the digital representation of the personality data of the user may be processed at the client device to configure at least one device providing a service to the user, wherein the at least one device may comprise the client device. The method performed by the client device further comprises sending, to the server, feedback characterizing the user, and receiving, from the server, a digital representation of second personality data of the user, wherein the second personality data of the user is computed using the neural network being updated based on the feedback. The digital representation of the second personality data of the user is processed at the client device to refine a configuration of the at least one device providing the service to the user. The feedback may include behavioral data reflecting behavior of the user monitored at the at least one device when using the service provided by the at least one device, wherein the behavioral data may be monitored using measurements performed by the at least one device providing the service to the user. The at least one device may comprise a vehicle, wherein the behavioral data may comprise data reflecting a driving behavior of the user. The personality data of the user may be computed prior to sending the request to the server, wherein the request may include an access code previously provided by the server to the user upon computing the personality data of the user, the access code allowing the user to access the digital representation of the personality data of the user from different client devices. The input obtained from the user may correspond to digital scores reflecting answers to questions regarding at least one of personality, goals and motivations of the user.

According to a third aspect, a computer program product is provided. The computer program product comprises program code portions for performing the method of at least one of the first aspect and the second aspect when the computer program product is executed on one or more computing devices (e.g., a processor or a distributed set of processors). The computer program product may be stored on a computer readable recording medium, such as a semiconductor memory, DVD, CD-ROM, and so on.

According to a fourth aspect, a server for enabling retrieval of a digital representation of personality data of a user by a client device from the server is provided, wherein the digital representation of the personality data is processed at the client device to provide a user-adapted service to the user. The server comprises at least one processor and at least one memory, wherein the at least one memory contains instructions executable by the at least one processor such that the server is operable to perform any of the method steps presented herein with respect to the first aspect.

According to a fifth aspect, a client device for enabling retrieval of a digital representation of personality data of a user from a server is provided. The client device comprises at least one processor and at least one memory, wherein the at least one memory contains instructions executable by the at least one processor such that the client device is operable to perform any of the method steps presented herein with respect to the second aspect.

According to a sixth aspect, there is provided a system comprising a server according to the fourth aspect and at least one client device according to the fifth aspect.

### Brief Description of the Drawings

Further details and advantages of the technique presented herein will be described with reference to exemplary implementations illustrated in the figures, in which:
- Figs. 1a and 1b: illustrate exemplary compositions of a server and a client device according to the present disclosure;
- Fig. 2: illustrates a method which may be performed by the server according to the present disclosure;
- Fig. 3: illustrates a method which may be performed by the client device according to the present disclosure;
- Fig. 4: illustrates an exemplary interaction between a user, the server and a client device (exemplified by a car) according to the present disclosure;
- Fig. 5: illustrates different connectivity options between a mobile terminal of the user, the car and the server according to the present disclosure;
- Figs. 6a and 6b: illustrate exemplary structures of the neural network according to the present disclosure;
- Fig. 7: illustrates an exemplary implementation which involves considering the driver's attention level to adapt settings of a vehicle according to the present disclosure; and
- Fig. 8: illustrates an exemplary implementation which involves considering body scan data of a user to provide a user-adapted service to the user according to the present disclosure.

### Detailed Description

In the following description, for purposes of explanation and not limitation, specific details are set forth in order to provide a thorough understanding of the present disclosure. It will be apparent to one skilled in the art that the present disclosure may be practiced in other implementations that depart from these specific details as long as they fall into the scope of the appended claims.

Those skilled in the art will further appreciate that the steps, services and functions explained herein below may be implemented using individual hardware circuitry, using software functioning in conjunction with a programmed micro-processor or general purpose computer, using one or more Application Specific Integrated Circuits (ASICs) and/or using one or more Digital Signal Processors (DSPs). It will also be appreciated that when the present disclosure is described in terms of a method, it may also be embodied in one or more processors and one or more memories coupled to the one or more processors, wherein the one or more memories are encoded with one or more programs that perform the steps, services and functions disclosed herein when executed by the one or more processors.

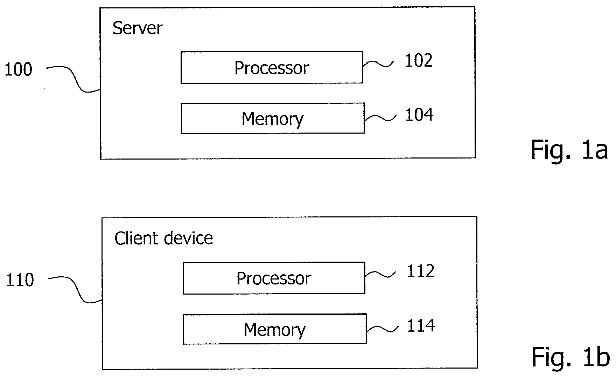

Figure 1a schematically illustrates an exemplary composition of a server 100 for enabling efficient retrieval of a digital representation of personality data of a user by a client device from the server 100, wherein the digital representation of the personality data is to be processed at the client device to provide a user-adapted service to the user. The server 100 comprises at least one processor 102 and at least one memory 104, wherein the at least one memory 104 contains instructions executable by the at least one processor 102 such that the request server 100 is operable to carry out the method steps described herein with reference to the "server".

It will be understood that the server 100 may be implemented on a physical computing unit or a virtualized computing unit, such as a virtual machine, for example. It will further be appreciated that the server 100 may not necessarily be implemented on a standalone computing unit, but may be implemented as components - realized in software and/or hardware - residing on multiple distributed computing units as well, such as in a cloud computing environment, for example.

Figure 1b schematically illustrates an exemplary composition of a client device 110 for enabling efficient retrieval of a digital representation of personality data of a user by the client device 110 from a server. The client device 110 comprises at least one processor 112 and at least one memory 114, wherein the at least one memory 114 contains instructions executable by the at least one processor 112 such that the request client device 110 is operable to carry out the method steps described herein with reference to the "client device".

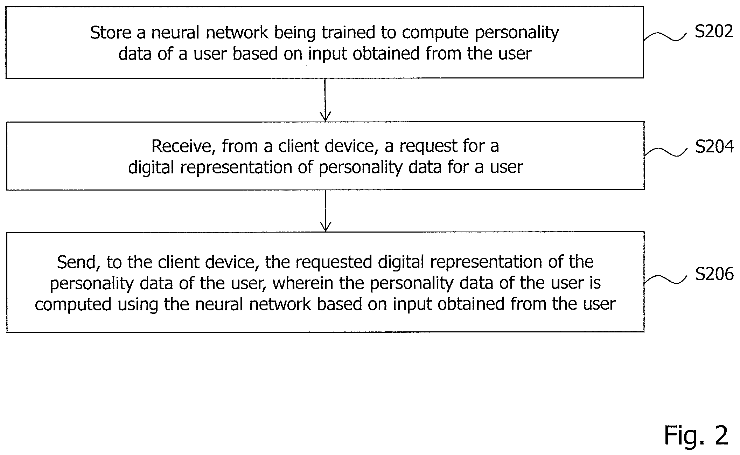

Figure 2 illustrates a method which may be performed by the server 100 according to the present disclosure. The method is dedicated to enabling efficient retrieval of a digital representation of personality data of a user by a client device (e.g., the client device 110) from the server 100. In the method, the server 100 may perform the steps described herein with reference to the "server" and, in line with the above description, in step S202, the server 100 may store a neural network being trained to compute personality data of a user based on input obtained from the user, in step S204, the server 100 may receive, from the client device, a request for a digital representation of personality data for a user and, in step S206, the server 100 may send, to the client device, the requested digital representation of the personality data of the user, wherein the personality data of the user is computed using the neural network based on input obtained from the user.

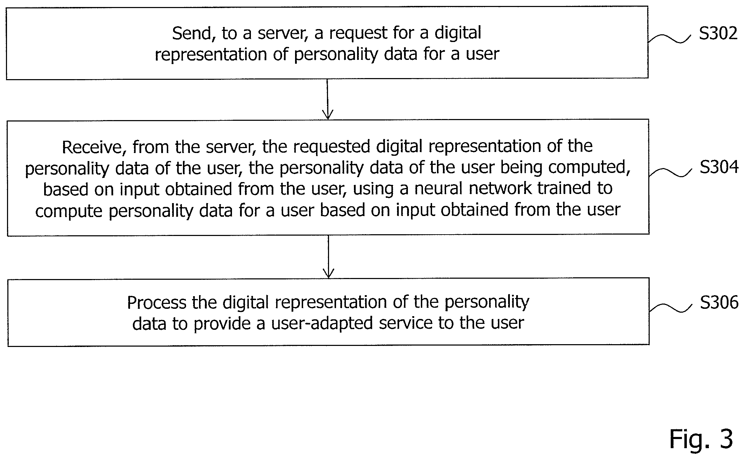

Figure 3 illustrates a method which may be performed by the client device 110 according to the present disclosure. The method is dedicated to enabling efficient retrieval of a digital representation of personality data of a user by the client device 110 from a server (e.g., the server 100). In the method, the client device 110 may perform the steps described herein with reference to the "client device" and, in line with the above description, in step S302, the client device 110 may send, to the server, a request for a digital representation of personality data for a user, in step S304, the client device 110 may receive, from the server, the requested digital representation of the personality data of the user, the personality data of the user being computed, based on input obtained from the user, using a neural network trained to compute personality data for a user based on input obtained from the user and, in step S306, the client device 110 may process the digital representation of the personality data to provide a user adapted service to the user.

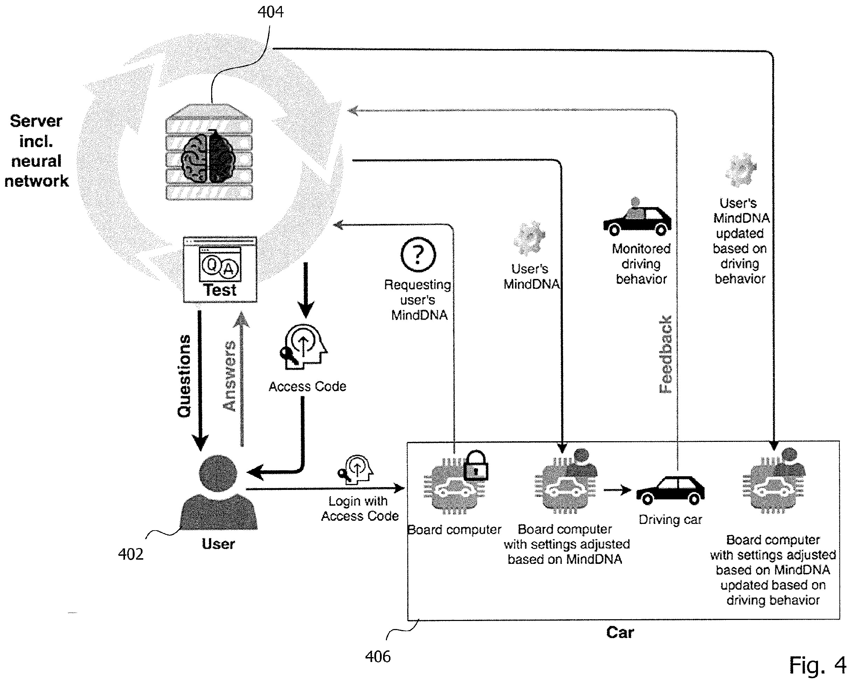

Figure 4 illustrates an exemplary interaction between a user 402, a server 404 storing a neural network being trained to compute personality data of users based on input obtained from the users, and a client device for retrieving a digital representation of personality data of the user 402 to provide a user-adapted service to the user 402, wherein, in the shown example, the client device is a car 406 which may be driven by the user 402. As shown in the figure, the user 402 may perform an automated personality test by answering questions, e.g., using a web interface or an app on his laptop or smartphone, to thereby provide input to the neural network stored at the server 404 based on which the neural network may compute personality data for the user 402. Instead of sending a digital representation of the personality data to the user 402, in the shown example, the server 404 provides an access code to the user 402 which can be used by the user 402 to access the personality data using different client devices, including the car 406. The user 402 may register or login at the car 406 (more specifically, at its board computer) with the access code and the car 406 may then request, using the access code, the digital representation of the user's personality data from the server 404 (in the figure, the personality data of the user is denoted as the user's "MindDNA").

Upon receiving the request from the car 406, the server 404 may return the user's personality data to the car 406, which may then configure its driving configuration (and, optionally, subcomponents of the car 406) in accordance with the personality data of the user 402, e.g., adapting the gas and brake reaction behavior of the car 406, to thereby provide a driving experience that is specifically adapted to the user's personality (e.g., risk-averse, risk-seeking, etc.). When the user 402 then drives the car 406, the car 406 may monitor the user's driving behavior, e.g., using sensors measuring the user's break reaction and intensity, and the car 406 may provide this information as feedback to the server 404, where the feedback may be processed to update (by training) the neural network to refine its capability of computing the personality data of the user 402. In response, the server 404 may send correspondingly updated personality data of the user 402 to the car 406 which may then use the digital representation of the updated personality data to refine the car configuration for a better alignment with the actual personality of the user 402. In sum, a system is therefore provided which may allow integrating retrieval and use of the user's personality data into an automated process to adapt the configuration of devices or services provided thereon in accordance with the preferences of the user derived his personality data, to thereby improve user experience.

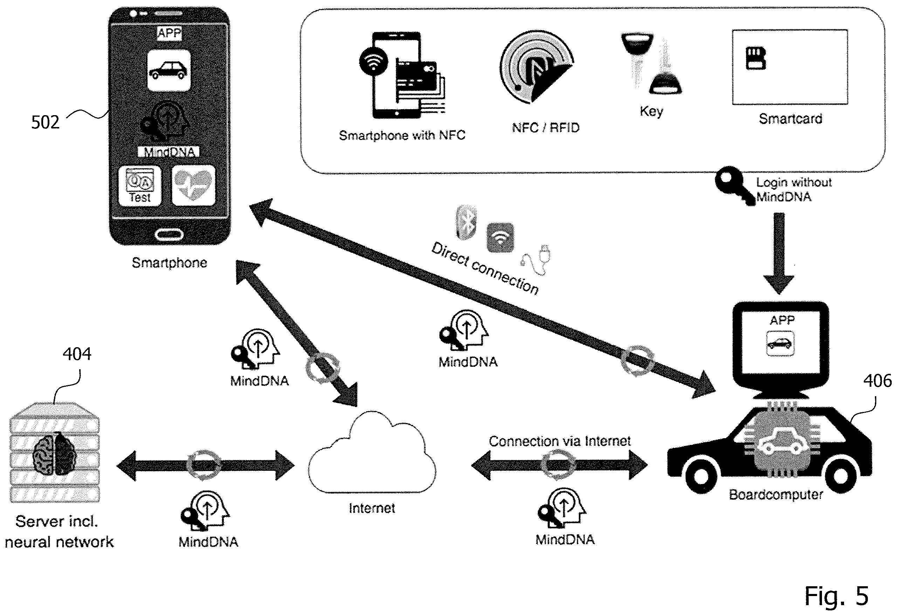

Figure 5 illustrates different connectivity options between a mobile terminal 502 (e.g., a smartphone) of the user 402, the car 406 and the server 404 in accordance with the present disclosure. In one variant, the car 406 may communicate with the server 404 directly via the Internet and, upon authentication of the user 402 with the car 406 (e.g., using a key, smartcard, NFC/RFID, a smartphone with NFC, fingerprint, or the like), the car 406 may request the personality data of the user (in Figure 5 again denoted as the user's "MindDNA") to improve the driving experience of the user 402. In another variant, when the user 402 carries the mobile terminal 502, the mobile terminal 502 may (e.g., using a dedicated app installed thereon) communicate with the server 404 via the Internet and request the personality data of the user 402. In this variant, the car 406 may communicate locally with the mobile terminal 502 (e.g., using Bluetooth, Wi-Fi or USB cable) and retrieve the personality data of the user from the mobile terminal 502. A direct connection between the car 406 and the mobile terminal 502 may additionally be used to exploit sensors installed at the mobile terminal 502 (e.g., gyroscope for movement and acceleration detection, GPS for movement and acceleration detection as well as detection of driving routes, or medical sensors measuring pulse, blood pressure, or the like) to supplement the feedback gathered by the car 406 itself (e.g., in relation to the user's driving behavior) to thereby provide additional feedback sensed by the mobile terminal 502 to the server 404 for updating the neural network based on the feedback, as described above.

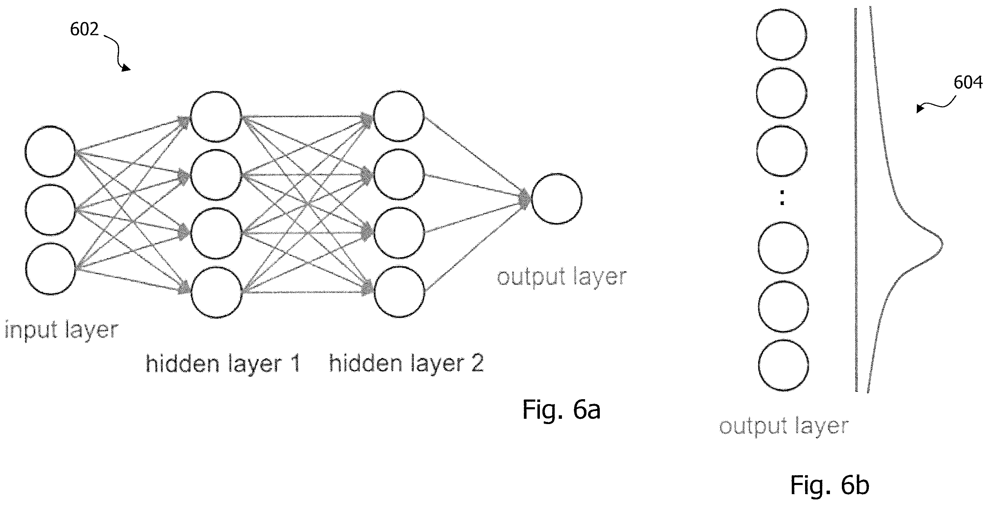

Figure 6a illustrates an exemplary structure of a neural network 602 in accordance with the present disclosure. The neural network 602 comprises an input layer, an output layer and two hidden layers. It will be understood that the neural network 602 shown in Figure 6a merely illustrates the structure of deep neural networks in general and that the actual number of nodes (at least in the input layer and the hidden layers) of the neural network 602 stored in the server 404 may be significantly higher than shown in the figure. As mentioned above, a test has been performed using the 30 most influential questions among a total number of 370 questions or more (taken from standard IPIP, HEXACO-60 and BFI-10 questions and, optionally, supplemented by further questions regarding goals and motivations of the user), resulting in 30 input nodes in the input layer of the neural network 602. In such a case, each of the hidden layers could be configured with 50 nodes, for example. Further, as shown in the figure, the neural network 602 may comprise a single output node in the output layer. In this case, the result value at the output node of the output layer may be representative of the value of one personality dimension (out of the Big Five) on which the neural network 602 has been trained. It will be understood that such structure of the neural network 602 is merely exemplary and that other structures are generally conceivable.

A more advanced structure of the neural network 602 comprises input nodes in accordance with the number of a full set of questions available, which may be taken from standard IPIP, HEXACO-60 and BFI-10 questions including further questions regarding goals and motivations of the user as well as still further questions on other psychological characteristics and/or preferences of the user not covered by the above questions, potentially adding up to several hundreds of questions, e.g., more than 600 questions. Such neural network 602 may thus have more than 600 input nodes, each corresponding to one of the questions of the full set of available questions, and the number of nodes of the hidden layers may be selected depending on the performance of the neural network 602. For example, the neural network 602 may comprise two hidden layers with 100 nodes each. Further, in the input layer, the above-mentioned more than 600 input nodes may be duplicated, wherein each duplicated input node may be used as a missing-question-indicator. The missing-question-indicators may be dichotomous, i.e., they may only have two values (e.g., 0 and 1) indicating whether the question of the corresponding (original) input node has been answered or not. Due to the duplicated input nodes, the input layer may comprise a total of more than 1200 input nodes.

The output layer of the more advanced neural network 602 may have a plurality of output nodes that together represent a probability curve for one personality dimension. If the scale used for the output in this personality dimension ranges from 0 to 10 and the number of output nodes is 50, for example, then each output node may be representative for a portion of the scale, i.e., corresponding to the portions 0-0.2, 0.2-0.4, 0.4-0.6, ... 9.8.10 of the scale. Instead of a single output value, such output layer may deliver a whole probability curve for the output value on this personality dimension. Figure 6b illustrates an exemplary output layer together with a corresponding probability curve 604. Such curve may allow determining where the output value most probably is (i.e., indicated by the peak of the curve) as well as determining the accuracy with which the neural network 602 calculates the result (i.e., indicated by the width of the curve). Using the advanced neural network 602, it may be possible to calculate the personality data of the user in the form of several probability curves (e.g., five probability curves corresponding to the Big Five) for an arbitrary number of answered questions, provided that the neural network 602 is trained separately for each dimension. In the initial state, in which no question has been answered yet, all missing-question-indicators may have the value of "missing" (e.g., 0). With every question which is then answered, an update of the output values may be calculated so that the width of the probability curves on the output layer becomes less with an increasing number of answered questions, so that the accuracy with which the neural network 602 calculates the result steadily increases.

Such structure of the neural network 602 may be particularly advantageous because it may allow iteratively selecting questions to be answered by the user next from the full set of questions, wherein, in each iteration, a next question may be selected depending on an answer of the user to the previous question, wherein, in each iteration, a next question may be selected as a question of the full set of questions which is determined to be most influential on an achievable result for computing personality data of the user. To this end, upon each answered question, the several (e.g., five) probability curves may be recalculated and, among the recalculated probability curves, the one which has the largest width (i.e., representing the probability curve currently having the at least accuracy) may be determined. As next question for the iteration, a question on this dimension may be selected to improve the accuracy on this dimension. In order to determine the most influential question, a degree according to which a change in the digital score input to the respective input node changes the probability curve (e.g., a degree in which the width of the curve changes) may be determined for each input node of the neural network 602. Based on this, the question associated with the input node for which the degree of change in the probability curve is determined to be highest may be selected as the most influential question for the respective iteration.

The advanced structure of the neural network 602 may also be advantageous because it may allow integrating feedback easily into the neural network. As described above, if the feedback represents a new input value which has not yet been input to the neural network 602, a new input node may simply be added to the neural network 602 and the new input value may be assigned to the new input node when training the neural network 602. In this way, any kind of new feedback may easily integrated into the network so that the neural network 602 may be refine its capability to compute personality data. As an implementation which reduces the computational complexity when adding a new input node, it may be conceivable that, when the network is trained to correlate the new input node with the other nodes of the network, only those nodes may be incorporated into the calculation which are determined to be most influential with respect to the optimally achievable result, to thereby avoid incorporating all nodes into the calculation. Also, it may be conceivable that, when the network is trained to correlate the new input node with the other nodes of the network, the number of layers being precalculated is limited (e.g., to 2 or 3) to avoid calculating all subsequent combinations of nodes, for example.

In the above description, the presented technique for efficient retrieval for a digital representation of personality data of a user has been exemplified in the context of adapting a vehicle's driving configuration, such as adapting the gas and brake reaction behavior of the vehicle to the personality of the user. In this case, the method described herein may also be denoted as a method for adapting a vehicle's driving configuration including an efficient retrieval of a digital representation of personality data of a user. It will be understood that adapting the gas and brake reaction behavior of the vehicle is just one example of adapting a vehicle's driving configuration and that, more generally, adapting the vehicle's driving configuration may comprise adapting any vehicle configuration that influences the driving behavior of the vehicle. Adapting the vehicle's driving configuration may as such comprise at least one of adapting a gas and brake reaction behavior of the vehicle, adapting chassis settings of the vehicle, adapting a driving mode of the vehicle, and adapting settings of an adaptive cruise control (ACC) of the vehicle, or the like, to the personality of the user. Adapting a driving mode of the vehicle may comprise setting an economy, comfort or sport mode to influence gas pedal and fuel consumption behavior of the vehicle depending on the driver's personality. If the personality data indicates that the driver tends to be risk-averse, for example, the driving mode may be set to economy or comfort, whereas for drivers that tend to have a risk-seeking personality, the driving mode may be set to sport mode. Adapting a drive mode of the vehicle may also comprise enabling/disabling an automatic four-wheel-drive (4WD) mode of the vehicle, for example. Adapting the settings of the ACC may comprise setting the distance to the vehicle ahead and/or the target driving speed, e.g., depending on the risk-averseness of the driver.

It will be understood that the technique presented herein may also be employed for other purposes in a vehicle context, such as to adapt the environmental conditions in the passenger cabin of the vehicle (or, more generally, of a transport means, as an adaptation of the environmental conditions in the passenger cabin may similarly apply to other means of transport, such as aircrafts, trains, etc.). In this case, the method described herein may also be denoted as a method for adapting an environmental condition in a passenger cabin of a transport means including an efficient retrieval of a digital representation of personality data of a user. Adapting an environmental condition in a passenger cabin of a transport means may comprise adapting at least one of adapting a temperature of the passenger cabin (e.g., by adapting the air condition settings for the passenger cabin), adapting an internal lighting of the passenger cabin, and adapting an oxygen level in the passenger cabin, or the like, to the personality of the user. Additionally or alternatively to adapting an environmental condition in the passenger cabin, the technique presented herein may also be employed to adapt user-specific settings regarding the passenger cabin. Adapting a user-specific setting regarding a passenger cabin of a transport means may comprise adapting at least one of adapting a seat configuration (e.g., seat height, seat position, seat massage settings, seat belt tensioning, etc.) for the user in the passenger cabin, and adapting equalizer settings of a sound system (e.g., increasing/decreasing basses or heights) provided to the user in the passenger cabin, or the like, to the personality of the user.

Any of the above adaptations of vehicle/transport means settings may - in addition to the adaptation to the personality of the user - also be performed in consideration of (or "based on"/"in accordance with") sensor data indicative of a user's attention level obtained in the passenger cabin. In other words, the client device may be configured to adapt at least one of the vehicle's driving configuration, the environmental conditions in the passenger cabin, and the user-specific settings regarding the passenger cabin not only in consideration of the digital representation of the personality data of the user, but also in consideration of sensor data indicative of a user's attention level. The sensor data indicative of the user's attention level may comprise data regarding at least one of the user's heartbeat, breath, tiredness, reaction time, and alcohol/drug level, for example. The sensor data may be collected by at least one sensor installed in the passenger cabin or in the mobile terminal of the user, for example.

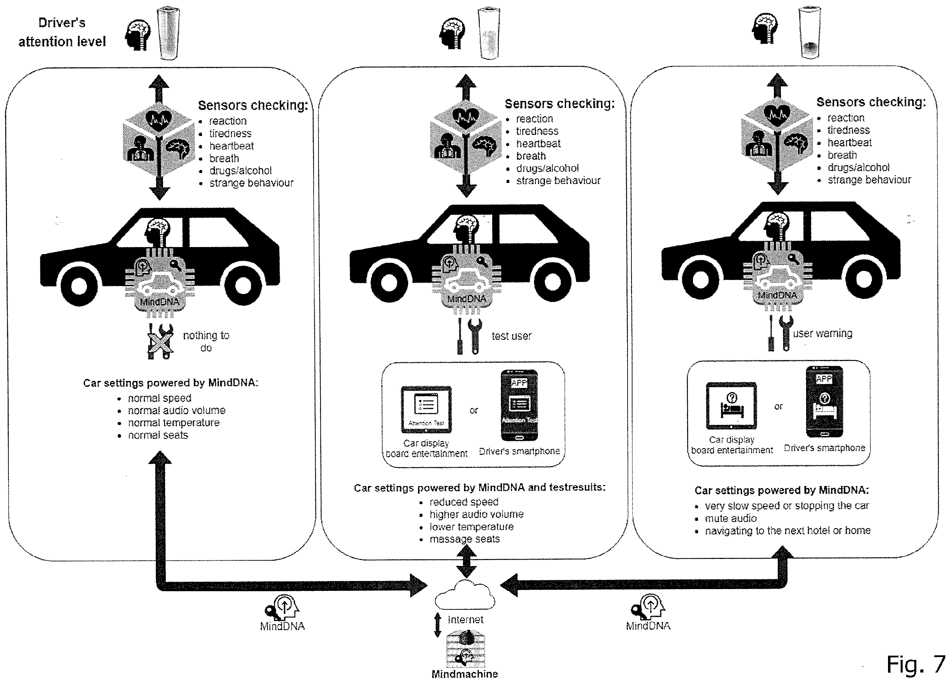

Figure 7 illustrates an exemplary implementation which involves considering the driver's attention level in combination with the driver's personality data in order to adapt the vehicle's driving configuration, the environmental conditions in the passenger cabin and/or the user-specific settings regarding the passenger cabin. The driver's attention level may be checked by corresponding sensors in terms of the user's reaction time, tiredness, heartbeat, breath, alcohol/drug level, or unusual behavior of the user, for example. In the left portion of the figure, the collected sensor data is indicative of a normal attention level of the user and, hence, the vehicle settings may remain at the normal levels (e.g., as adapted to the driver's personality or "MindDNA"), including the speed, audio volume, temperature and seat settings, for example. In the middle portion of the figure, the sensor data is indicative of a reduced attention level of the driver and the vehicle settings may thus be changed to reduced speed, higher audio volume, lower temperature settings, including turning on seat massage features, in order to refresh the driver's attention again. Optionally, attention tests may be performed, such as requesting the driver to provide a voice-based response in a question/answer scheme, for example, and the results of the attention tests may be considered in adapting the above-mentioned settings. In the right portion of the figure, on the other hand, the sensor data is indicative of a very low driver's attention level and, therefore, a user warning may be provided and the vehicle settings may be adapted accordingly, e.g., to a very slow speed (and forcing to stop the vehicle at the next stopping opportunity, for example), to muted audio and/or to provide directions to the next hotel by a navigation system, for example.

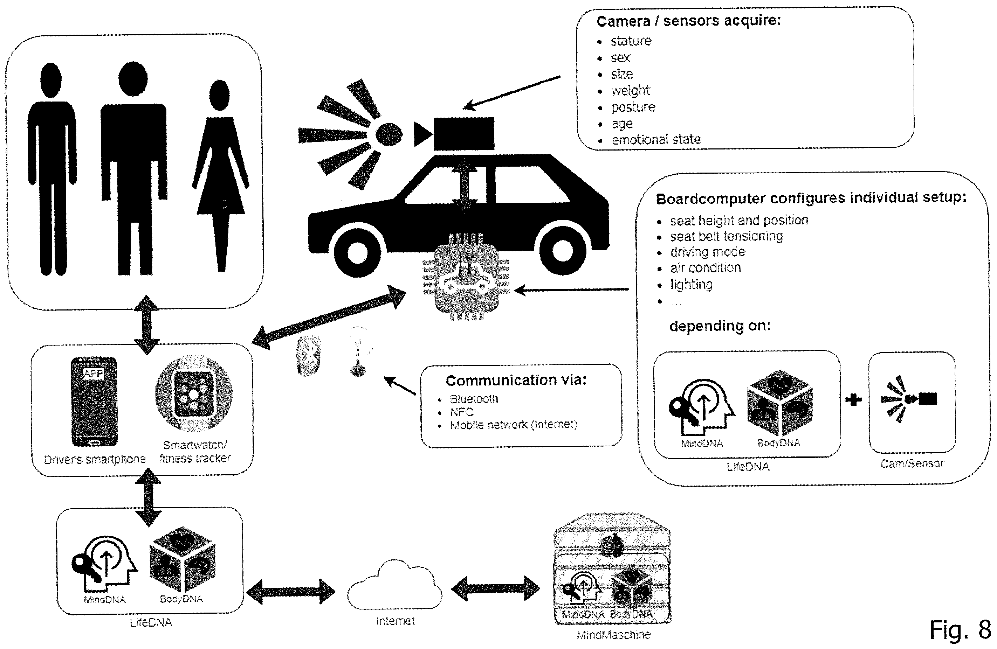

In order to provide a user-adapted service to the user, as described above (e.g., by adapting at least one of the vehicle's driving configuration, the environmental conditions in the passenger cabin, and the user-specific settings regarding the passenger cabin), the client device may further consider body scan data indicative of (e.g., physical) characteristics of the user derivable by scanning (e.g., at least a portion of) the user's body prior to providing the user-adapted service to the user (e.g., prior to the user driving the vehicle). The user characteristics which are derivable by scanning the user's body may include at least one of the user's size, weight, sex, age, stature, posture, and emotional state, for example. The body scan data may be obtained by a camera or voice recorder (e.g., of the mobile terminal of the user, or installed at the vehicle/transport means) acquiring one or more images or speech signals of the user, wherein body/face/voice recognition techniques may be employed to scan the user's body and derive the user characteristics mentioned above. The client device may thus be configured to provide a user-adapted service not only in consideration of the digital representation of the personality data of the user, but also in consideration of (or "based on"/"in accordance with") the body scan data. Figure 8 illustrates an exemplary implementation which involves considering a driver's body scan data (e.g., obtained by the driver's mobile terminal, such as the driver's smartphone, smartwatch or fitness tracker, prior to entering the vehicle) in combination with the personality data of the driver in order to adapt the vehicle's driving configuration, the environmental conditions in the passenger cabin and/or the user-specific settings regarding the passenger cabin accordingly. In the figure, the body scan data is denoted as "BodyDNA", which in combination with the "MindDNA", forms the so-called "LifeDNA". It will be understood that the obtained body scan data may also be used to provide feedback characterizing the user to update the neural network, as described above.

In another vehicle-related use case, which does not form part of the claimed invention, the technique presented herein may also be used to determine a vehicle configuration that is adapted to the personality of the user prior to manufacturing the vehicle, wherein the vehicle may then be manufactured based on (or "in accordance with") the determined vehicle configuration. The vehicle may be manufacturable in different configuration options (e.g., as offered by a vehicle manufacturer), such as with different motor options each having a different motor power, drive technology options (e.g., support of two-wheel-drive (2WD) or 4WD technology), chassis options, different drive mode options, support of ACC, etc., and when a new vehicle is to be manufactured for the user, the vehicle configuration may be determined to be specifically adapted to the personality of the user. For example, if the personality data indicates that the user tends to be risk-averse, the determined vehicle configuration may comprise a selection of a motor having a lower power as compared to a vehicle configuration determined for a user whose personality data indicates a risk-seeking personality. Based on the determined vehicle configuration, the vehicle may then be manufactured accordingly. As such, in line with the above description, it may also be envisaged a method for vehicle manufacturing, which does not form part of the claimed invention, including an efficient retrieval of a digital representation of personality data of a user by a client device from a server, the digital representation of the personality data being processed at the client device to provide a vehicle configuration adapted to the personality of the user. The method may comprise sending, from the client device to the server, a request for a digital representation of personality data for a user, receiving, by the client device from the server, the requested digital representation of the personality data of the user, the personality data of the user being computed, based on input obtained from the user, using a neural network trained to compute personality data for a user based on input obtained from the user, processing the digital representation of the personality data to determine a vehicle configuration which is adapted to the personality of the user, and manufacturing the vehicle based on the determined vehicle configuration. In the manufacturing process of the vehicle, it will be understood that the determined vehicle configuration may also affect the manufacturing of vehicle parts needed for the manufacturing of the vehicle. For example, manufacturing the vehicle may comprise manufacturing one or more vehicle parts to be used for manufacturing the vehicle, wherein the vehicle parts are manufactured (e.g., using a 3D printer) in accordance with the determined vehicle configuration.

In all of the above-described examples and use cases, when it is referred to "adapting" a configuration or setting "to the personality of a user", such adaptation is implemented using mappings that map a given characteristic of the user's personality (as indicated by the digital representation of the personality data of the user) to a particular configuration or setting of the corresponding device/apparatus (e.g., vehicle, transport means, as described above). As said, for example, if the personality data indicates that a driver tends to be risk-averse, the driving mode of a vehicle may be set to economy or comfort, whereas for drivers that tend to have a risk-seeking personality, the driving mode may be set to sport mode. Such mappings may be predefined for each possible personality characteristic-configuration/setting combination and, depending on the obtained personality data of the user, the configuration or setting of the device/apparatus may be adapted accordingly. The personality characteristic of the user may correspond to a value of a personality dimension (e.g., out of the Big Five) output by the neural network, as described above, for example.

It is believed that the advantages of the technique presented herein will be fully understood from the foregoing description, and it will be apparent that various changes may be made in the form, constructions and arrangement of the exemplary aspects thereof without departing from the scope of the disclosure or without sacrificing all of its advantageous effects. Because the technique presented herein can be varied in many ways, it will be recognized that the disclosure is limited only by the scope of the claims that follow.

## Claims

1. A method including a retrieval of a digital representation of personality data of a user (402) by a client device (502; 406) from a server (404), the digital representation of the personality data being processed at the client device (406) to provide a user-adapted service to the user (402), the method being performed by the server (404) and comprising:
storing (S202) a neural network (602) trained to compute personality data of a user (402) based on input obtained from the user (402);
receiving (S204), from the client device (502; 406), a request for a digital representation of personality data for a user (402); and
sending (S206), to the client device (502; 406), the requested digital representation of the personality data of the user (402),
wherein the digital representation of the personality data of the user (402) is processed at the client device (502; 406) to adapt a configuration of at least one device (406) providing a service to the user (402), wherein adapting the configuration of the at least one device (406) includes one of:
- adapting a vehicle's driving configuration, wherein the at least one device comprises a vehicle (406) and wherein the digital representation of the personality data of the user (402) is processed at the client device (502; 406) to adapt a driving configuration of the vehicle (406) to a personality of the user (402),
- adapting an environmental condition in a passenger cabin of a transport means (406), wherein the at least one device comprises the transport means (406) and wherein the digital representation of the personality data of the user (402) is processed at the client device (502; 406) to adapt an environmental condition in the passenger cabin of the transport means (406) to a personality of the user (402),
- adapting a user-specific setting regarding a passenger cabin of a transport means (406), wherein the at least one device comprises the transport means (406) and wherein the digital representation of the personality data of the user (402) is processed at the client device (502; 406) to adapt a user-specific setting regarding the passenger cabin of the transport means (406) to a personality of the user (402),
wherein adapting the configuration of the at least one device (406) to the personality of the user (402) is implemented using mappings that map characteristics of the personality of the user (402) as indicated by the digital representation of the personality data of the user (402) to configurations of the at least one device (406),
wherein the personality data of the user (402) is computed using the neural network (602) based on input obtained from the user (402), wherein the method comprises receiving feedback characterizing the user (402), the feedback being indicative of the personality of the user (402), and the personality data of the user (402) being indicative of at least one of psychological characteristics of the user (402) and preferences of the user (402);
updating the neural network (602) based on the feedback, wherein updating the neural network includes training the neural network based on the feedback; and
sending, to the client device (502; 406), a digital representation of second personality data of the user (402), wherein the second personality data of the user (402) is computed using the updated neural network (602),
wherein the digital representation of the second personality data of the user (402) is processed at the client device (502; 406) to refine the configuration of the at least one device (406) providing the user-adapted service to the user (402).

2. The method of claim 1, wherein the at least one device (406) comprises the client device (406).

3. The method of claim 1 or 2, wherein the feedback is gathered at the client device (110).

4. The method of any one of claims 1 to 3, wherein the feedback includes behavioral data reflecting behavior of the user (402) monitored at the at least one device (406) when using the service provided by the at least one device (406), and, optionally:
wherein the behavioral data is monitored using measurements performed by the at least one device (406) providing the service to the user (402).

5. The method of claim 4, wherein the at least one device (406) comprises a vehicle and wherein the behavioral data comprises data reflecting a driving behavior of the user (402).

6. The method of any one of claims 1 to 5, wherein the input obtained from the user corresponds to digital scores reflecting answers to questions regarding the personality of the user (402), and, optionally:
wherein each digital score is used as input to a separate input node of the neural network (602) when computing the personality data of the user (402) using the neural network (602), and/or
wherein the questions regarding the personality of the user (402) correspond to questions of at least one of:
an International Personality Item Pool, IPIP,
a HEXACO-60 pool,
a Big-Five-Inventory-10, BFI-10, pool,
questions on psychological characteristics of the user (402), and
questions on preferences of the user (402).

7. The method of claim 6, wherein the questions correspond to questions selected from a set of questions representative of an optimally achievable result of computing personality data of a user (402), wherein the selected questions correspond to questions of the set of questions which are determined to be most influential with respect to the optimally achievable result, and, optionally:
wherein the number of the selected questions is less than 10% of the number of questions included in the set of questions.

8. The method of claim 7, wherein the questions are selected from the set of questions based on correlating results achievable by each single question of the set of questions with the optimally achievable result and selecting questions from the set of questions which have a highest correlation with the optimally achievable result, or
wherein the questions are selected iteratively from the set of questions, wherein, in each iteration, a next question is selected depending on an answer of the user to a previous question, wherein, in each iteration, the next question is selected as a question of the set of questions which is determined to be most influential on an achievable result for computing personality data of the user, and, optionally:
wherein the neural network (602) comprises a plurality of output nodes representative of a probability curve (604) of a result of the personality data of the user (402), wherein determining the most influential question of the set of questions as the next question of the respective iteration includes determining, for each input node of the neural network (602), a degree according to which a change in the digital score input to the respective input node of the neural network (602) changes the probability curve (604).

9. The method of any one of claims 1 to 8, wherein:
(a) the personality data of the user (402) is computed prior to receiving the request from the client device (502; 406) and wherein the request includes an access code previously provided by the server (404) to the user (402) upon computing the personality data of the user (402), the access code allowing the user (402) to access the digital representation of the personality data of the user (402) from different client devices (502; 406); or
(b) when the at least one device (406) comprises a transport means, providing the user-adapted service to the user (402) is further performed in consideration of sensor data indicative of an attention level of the user (402) obtained in a passenger cabin of the transport means (406); or
(c) providing the user-adapted service to the user (402) is further performed in consideration of body scan data indicative of characteristics of the user (402) derivable by scanning at least a portion of the body of the user (402).

10. A method including a retrieval of a digital representation of personality data of a user (402) by a client device (502; 406) from a server (404), the method being performed by the client device (502; 406) and comprising:
sending (S302), to the server (404), a request for a digital representation of personality data for a user (402);
receiving (S304), from the server (404), the requested digital representation of the personality data of the user (402), the personality data of the user (402) being computed, based on input obtained from the user (402), using a neural network (602) trained to compute personality data for a user (402) based on input obtained from the user (402); and
processing (S306) the digital representation of the personality data to provide a user-adapted service to the user (402),
wherein the digital representation of the personality data of the user (402) is processed at the client device (502; 406) to adapt a configuration of at least one device (406) providing a service to the user (402), wherein adapting the configuration of the at least one device (406) includes one of:
- adapting a vehicle's driving configuration, wherein the at least one device comprises a vehicle (406) and wherein the digital representation of the personality data of the user (402) is processed at the client device (502; 406) to adapt a driving configuration of the vehicle (406) to a personality of the user (402),
- adapting an environmental condition in a passenger cabin of a transport means (406), wherein the at least one device comprises the transport means (406) and wherein the digital representation of the personality data of the user (402) is processed at the client device (502; 406) to adapt an environmental condition in the passenger cabin of the transport means (406) to a personality of the user (402),
- adapting a user-specific setting regarding a passenger cabin of a transport means (406), wherein the at least one device comprises the transport means (406) and wherein the digital representation of the personality data of the user (402) is processed at the client device (502; 406) to adapt a user-specific setting regarding the passenger cabin of the transport means (406) to a personality of the user (402),
wherein adapting the configuration of the at least one device (406) to the personality of the user (402) is implemented using mappings that map characteristics of the personality of the user (402) as indicated by the digital representation of the personality data of the user (402) to configurations of the at least one device (406),
wherein the method further comprises:
sending, to the server (404), feedback characterizing the user (402), the feedback being indicative of the personality of the user (402), and the personality data of the user (402) being indicative of at least one of psychological characteristics of the user (402) and preferences of the user (402); and
receiving, from the server (404), a digital representation of second personality data of the user (402), wherein the second personality data of the user (402) is computed using the neural network (602) being updated based on the feedback, wherein updating the neural network includes training the neural network based on the feedback,
wherein the digital representation of the second personality data of the user (402) is processed at the client device (502; 406) to refine the configuration of the at least one device (406) providing the user-adapted service to the user (402).

11. A computer program product comprising program code portions for performing the method of any one of claims 1 to 10 when the computer program product is executed on one or more computing units.

12. A computer readable recording medium having stored thereon the computer program product of claim 11.

13. A server (100; 404) enabling retrieval of a digital representation of personality data of a user (402) by a client device (502; 406) from the server (404), the digital representation of the personality data being processed at the client device (502; 406) to provide a user-adapted service to the user (402), the server (404) comprising at least one processor (102) and at least one memory (104), the at least one memory (104) containing instructions executable by the at least one processor (102) such that the server (404) is operable to perform the method of any one of claims 1 to 9.

14. A client device (110; 502; 406) enabling retrieval of a digital representation of personality data of a user (402) from a server (404), the client device (110; 502; 406) comprising at least one processor (112) and at least one memory (114), the at least one memory (114) containing instructions executable by the at least one processor (112) such that the client device (110; 502; 406) is operable to perform the method of claim 10.

15. A system comprising a server (100; 404) according to claim 13 and at least one client device (110; 502; 406) according to claim 14.

## Patentansprüche

1. Verfahren umfassend ein Abrufen einer digitalen Repräsentation von Persönlichkeitsdaten eines Benutzers (402) durch eine Client-Einrichtung (502; 406) von einem Server (404), wobei die digitale Repräsentation der Persönlichkeitsdaten an der Client-Einrichtung (406) verarbeitet wird, um dem Benutzer (402) einen benutzerangepassten Dienst bereitzustellen, wobei das Verfahren von dem Server (404) durchgeführt wird und umfasst:
Speichern (S202) eines neuronalen Netzes (602), das trainiert wurde, um Persönlichkeitsdaten eines Benutzers (402) basierend auf Eingaben, die von dem Benutzer (402) erhalten werden, zu berechnen;
Empfangen (S204), von der Client-Einrichtung (502; 406), einer Anfrage nach einer digitalen Repräsentation von Persönlichkeitsdaten für einen Benutzer (402); und
Senden (S206), an die Client-Einrichtung (502; 406), der angefragten digitalen Repräsentation der Persönlichkeitsdaten des Benutzers (402),
wobei die digitale Repräsentation der Persönlichkeitsdaten des Benutzers (402) an der Client-Einrichtung (502; 406) verarbeitet wird, um eine Konfiguration zumindest einer Vorrichtung (406) anzupassen, die einen Dienst für den Benutzer (402) bereitstellt, wobei das Anpassen der Konfiguration der zumindest einen Vorrichtung (406) eines umfasst aus:
- Anpassen einer Fahrkonfiguration eines Fahrzeugs, wobei die zumindest eine Vorrichtung ein Fahrzeug (406) umfasst und wobei die digitale Repräsentation der Persönlichkeitsdaten des Benutzers (402) an der Client-Einrichtung (502; 406) verarbeitet wird, um eine Fahrkonfiguration des Fahrzeugs an eine Persönlichkeit des Benutzers (402) anzupassen,
- Anpassen einer Umgebungsbedingung in einem Fahrgastraum eines Transportmittels (406), wobei die zumindest eine Vorrichtung das Transportmittel (406) umfasst und wobei die digitale Repräsentation der Persönlichkeitsdaten des Benutzers (402) an der Client-Einrichtung (502; 406) verarbeitet wird, um eine Umgebungsbedingung in dem Fahrgastraum des Transportmittels (406) an eine Persönlichkeit des Benutzers (402) anzupassen,
- Anpassen einer benutzerspezifischen Einstellung bezüglich eines Fahrgastraums eines Transportmittels (406), wobei die zumindest eine Vorrichtung das Transportmittel (406) umfasst und wobei die digitale Repräsentation der Persönlichkeitsdaten des Benutzers (402) an der Client-Einrichtung (502; 406) verarbeitet wird, um eine benutzerspezifische Einstellung bezüglich des Fahrgastraums des Transportmittels (406) an eine Persönlichkeit des Benutzers (402) anzupassen,
wobei das Anpassen der Konfiguration der zumindest einen Vorrichtung (406) an die Persönlichkeit des Benutzers (402) unter Verwendung von Mappings implementiert wird, die Eigenschaften der Persönlichkeit des Benutzers (402), wie angegeben durch die digitale Repräsentation der Persönlichkeitsdaten des Benutzers (402), auf Konfigurationen der zumindest einen Vorrichtung (406) abbilden,
wobei die Persönlichkeitsdaten des Benutzers (402) unter Verwendung des neuronalen Netzes (602) basierend auf Eingaben, die von dem Benutzer (402) erhalten werden, berechnet werden, wobei das Verfahren ein Empfangen von Feedback, das den Benutzer charakterisiert (402), umfasst, wobei das Feedback auf die Persönlichkeit des Benutzers (402) hinweist, und die Persönlichkeitsdaten des Benutzers (402) auf zumindest eines aus psychologischen Eigenschaften des Benutzers (402) und Präferenzen des Benutzers (402) hinweisen;
Aktualisieren des neuronalen Netzes (602) basierend auf dem Feedback, wobei das Aktualisieren des neuronalen Netzes ein Trainieren des neuronalen Netzes basierend auf dem Feedback umfasst; und
Senden, an die Client-Einrichtung (502; 406), einer digitalen Repräsentation zweiter Persönlichkeitsdaten des Benutzers (402), wobei die zweiten Persönlichkeitsdaten des Benutzers (402) unter Verwendung des aktualisierten neuronalen Netzes (602) berechnet werden,
wobei die digitale Repräsentation der zweiten Persönlichkeitsdaten des Benutzers (402) an der Client-Einrichtung (502; 406) verarbeitet wird, um die Konfiguration der zumindest einen Vorrichtung (406), die den Dienst für den Benutzer (402) bereitstellt, zu verfeinern.

2. Verfahren nach Anspruch 1, wobei die zumindest eine Vorrichtung (406) die Client-Einrichtung (406) umfasst.

3. Verfahren nach Anspruch 1 oder 2, wobei das Feedback an der Client-Einrichtung (110) gesammelt wird.

4. Verfahren nach einem der Ansprüche 1 bis 3, wobei das Feedback Verhaltensdaten umfasst, die ein Verhalten des Benutzers (402) widerspiegeln, das an der zumindest einen Vorrichtung (406) beobachtet wird, wenn er den von der zumindest einen Vorrichtung (406) bereitgestellten Dienst verwendet, und optional:
wobei die Verhaltensdaten unter Verwendung von Messungen beobachtet werden, die von der zumindest einen Vorrichtung (406), die den Dienst für den Benutzer (402) bereitstellt, durchgeführt werden.

5. Verfahren nach Anspruch 4, wobei die zumindest eine Vorrichtung (406) ein Fahrzeug umfasst und wobei die Verhaltensdaten Daten umfassen, die ein Fahrverhalten des Benutzers (402) widerspiegeln.

6. Verfahren nach einem der Ansprüche 1 bis 5, wobei die von dem Benutzer erhaltenen Eingaben digitalen Werten entsprechen, die Antworten auf Fragen bezüglich der Persönlichkeit des Benutzers (402) widerspiegeln, und, optional:
wobei jeder digitale Wert als Eingabe für einen separaten Eingangsknoten des neuronalen Netzes (602) verwendet wird, wenn die Persönlichkeitsdaten des Benutzers unter Verwendung des neuronalen Netzes (602) berechnet werden, und/oder
wobei die Fragen bezüglich der Persönlichkeit des Benutzers (402) Fragen entsprechen aus zumindest einem aus:
einem International Personality Item Pool, IPIP,
einem HEXACO-60-Pool,
einem Big-Five-Inventory-10-, BFI-10-, Pool,
Fragen zu psychologischen Eigenschaften des Benutzers (402), und
Fragen zu Präferenzen des Benutzers (402).

7. Verfahren nach Anspruch 6, wobei die Fragen Fragen entsprechen, die aus einem Satz von Fragen gewählt sind, der repräsentativ ist für ein optimal erreichbares Ergebnis der Berechnung von Persönlichkeitsdaten eines Benutzers (402), wobei die gewählten Fragen Fragen aus dem Satz von Fragen entsprechen, die als am einflussreichsten in Bezug auf das optimal erreichbare Ergebnis bestimmt sind, und, optional:
wobei die Anzahl der gewählten Fragen weniger als 10 % der Anzahl der in dem Satz von Fragen enthaltenen Fragen beträgt.

8. Verfahren nach Anspruch 7, wobei die Fragen aus dem Satz von Fragen basierend auf einem Korrelieren von Ergebnissen, die durch jede einzelne Frage des Satzes von Fragen erreichbar sind, mit dem optimal erreichbaren Ergebnis und einem Wählen von Fragen aus dem Satz von Fragen, die eine höchste Korrelation mit dem optimal erreichbaren Ergebnis aufweisen, gewählt werden, oder
wobei die Fragen iterativ aus dem Satz von Fragen gewählt werden, wobei in jeder Iteration eine nächste Frage in Abhängigkeit von einer Antwort des Benutzers auf eine vorherige Frage gewählt wird, wobei in jeder Iteration die nächste Frage als eine Frage aus dem Satz von Fragen gewählt wird, die als am einflussreichsten auf ein erreichbares Ergebnis zur Berechnung von Persönlichkeitsdaten des Benutzers bestimmt wird, und optional:
wobei das neuronale Netz (602) eine Vielzahl von Ausgangsknoten umfasst, die repräsentativ sind für eine Wahrscheinlichkeitskurve (604) eines Ergebnisses der Persönlichkeitsdaten des Benutzers (402), wobei das Bestimmen der einflussreichsten Frage des Satzes von Fragen als die nächste Frage der jeweiligen Iteration ein Bestimmen, für jeden Eingangsknoten des neuronalen Netzes (602), eines Grades umfasst, gemäß dem eine Änderung in dem digitalen Wert, der in den jeweiligen Eingangsknoten des neuronalen Netzes (602) eingegeben wird, die Wahrscheinlichkeitskurve (604) ändert.

9. Verfahren nach einem der Ansprüche 1 bis 8, wobei:
(a) die Persönlichkeitsdaten des Benutzers (402) vor dem Empfangen der Anfrage von der Client-Einrichtung (502; 406) berechnet werden und wobei die Anfrage einen Zugangscode umfasst, der dem Benutzer (402) zuvor von dem Server (404) beim Berechnen der Persönlichkeitsdaten des Benutzers (402) bereitgestellt wurde, wobei der Zugangscode dem Benutzer (402) ermöglicht, Zugang zu der digitalen Repräsentation der Persönlichkeitsdaten des Benutzers (402) von verschiedenen Client-Einrichtungen (502; 406) aus zu erhalten; oder
(b) wenn die zumindest eine Vorrichtung (406) ein Transportmittel umfasst, das Bereitstellen des benutzerangepassten Diensts für den Benutzer (402) ferner unter Berücksichtigung von Sensordaten durchgeführt wird, die auf einen in dem Fahrgastraum des Transportmittels (406) bestimmten Aufmerksamkeitsgrad des Benutzers (402) hinweisen; oder
(c) das Bereitstellen des benutzerangepassten Diensts für den Benutzer (402) ferner unter Berücksichtigung von Körperscandaten durchgeführt wird, die auf Eigenschaften des Benutzers (402) hinweisen, die durch Scannen zumindest eines Teils des Körpers des Benutzers (402) ableitbar sind.

10. Verfahren umfassend ein Abrufen einer digitalen Repräsentation von Persönlichkeitsdaten eines Benutzers (402) durch eine Client-Einrichtung (502; 406) von einem Server (404), wobei das Verfahren durch die Client-Einrichtung (502; 406) durchgeführt wird und umfasst:
Senden (S302), an den Server (404), einer Anfrage nach einer digitalen Repräsentation von Persönlichkeitsdaten für einen Benutzer (402);
Empfangen (S304), von dem Server (404), der angefragten digitalen Repräsentation der Persönlichkeitsdaten des Benutzers (402), wobei die Persönlichkeitsdaten des Benutzers (402) basierend auf Eingaben, die von dem Benutzer (402) erhalten werden, unter Verwendung eines neuronalen Netzes (602) berechnet werden, das trainiert ist, um Persönlichkeitsdaten für einen Benutzer (402) basierend auf Eingaben, die von dem Benutzer (402) erhalten werden, zu berechnen; und
Verarbeiten (S306) der digitalen Repräsentation der Persönlichkeitsdaten, um dem Benutzer (402) einen benutzerangepassten Dienst bereitzustellen,
wobei die digitale Repräsentation der Persönlichkeitsdaten des Benutzers (402) an der Client-Einrichtung (502; 406) verarbeitet wird, um eine Konfiguration zumindest einer Vorrichtung (406) anzupassen, die einen Dienst für den Benutzer (402) bereitstellt, wobei das Anpassen der Konfiguration der zumindest einen Vorrichtung (406) eines umfasst aus:
- Anpassen einer Fahrkonfiguration eines Fahrzeugs, wobei die zumindest eine Vorrichtung ein Fahrzeug (406) umfasst und wobei die digitale Repräsentation der Persönlichkeitsdaten des Benutzers (402) an der Client-Einrichtung (502; 406) verarbeitet wird, um eine Fahrkonfiguration des Fahrzeugs an eine Persönlichkeit des Benutzers (402) anzupassen,
- Anpassen einer Umgebungsbedingung in einem Fahrgastraum eines Transportmittels (406), wobei die zumindest eine Vorrichtung das Transportmittel (406) umfasst und wobei die digitale Repräsentation der Persönlichkeitsdaten des Benutzers (402) an der Client-Einrichtung (502; 406) verarbeitet wird, um eine Umgebungsbedingung in dem Fahrgastraum des Transportmittels (406) an eine Persönlichkeit des Benutzers (402) anzupassen,
- Anpassen einer benutzerspezifischen Einstellung bezüglich eines Fahrgastraums eines Transportmittels (406), wobei die zumindest eine Vorrichtung das Transportmittel (406) umfasst und wobei die digitale Repräsentation der Persönlichkeitsdaten des Benutzers (402) an der Client-Einrichtung (502; 406) verarbeitet wird, um eine benutzerspezifische Einstellung bezüglich des Fahrgastraums des Transportmittels (406) an eine Persönlichkeit des Benutzers (402) anzupassen,
wobei das Anpassen der Konfiguration der zumindest einen Vorrichtung (406) an die Persönlichkeit des Benutzers (402) unter Verwendung von Mappings implementiert wird, die Eigenschaften der Persönlichkeit des Benutzers (402), wie angegeben durch die digitale Repräsentation der Persönlichkeitsdaten des Benutzers (402), auf Konfigurationen der zumindest einen Vorrichtung (406) abbilden,
wobei das Verfahren ferner umfasst:
Senden, an den Server (404), von Feedback, das den Benutzer (402) charakterisiert, wobei das Feedback auf die Persönlichkeit des Benutzers (402) hinweist, und die Persönlichkeitsdaten des Benutzers (402) auf zumindest eines aus psychologischen Eigenschaften des Benutzers (402) und Präferenzen des Benutzers (402) hinweisen; und
Empfangen, von dem Server (404), einer digitalen Repräsentation zweiter Persönlichkeitsdaten des Benutzers (402), wobei die zweiten Persönlichkeitsdaten des Benutzers (402) unter Verwendung des neuronalen Netzes (602) berechnet werden, das basierend auf dem Feedback aktualisiert wird, wobei das Aktualisieren des neuronalen Netzes ein Trainieren des neuronalen Netzes basierend auf dem Feedback umfasst,
wobei die digitale Repräsentation der zweiten Persönlichkeitsdaten des Benutzers (402) an der Client-Einrichtung (502; 406) verarbeitet wird, um die Konfiguration der zumindest einen Vorrichtung (406), die den Dienst für den Benutzer (402) bereitstellt, zu verfeinern.

11. Computerprogrammprodukt mit Programmcodeabschnitten zur Durchführung des Verfahrens nach einem der Ansprüche 1 bis 10, wenn das Computerprogrammprodukt auf einer oder mehreren Recheneinheiten ausgeführt wird.

12. Computerlesbares Aufzeichnungsmedium, auf dem das Computerprogrammprodukt nach Anspruch 11 gespeichert ist.

13. Server (100; 404), der ein Abrufen einer digitalen Repräsentation von Persönlichkeitsdaten eines Benutzers (402) durch eine Client-Einrichtung (502; 406) von dem Server (404) ermöglicht, wobei die digitale Repräsentation der Persönlichkeitsdaten an der Client-Einrichtung (502; 406) verarbeitet wird, um dem Benutzer (402) einen benutzerangepassten Dienst bereitzustellen, wobei der Server (404) zumindest einen Prozessor (102) und zumindest einen Speicher (104) umfasst, wobei der zumindest eine Speicher (104) Instruktionen enthält, die von dem zumindest einen Prozessor (102) ausführbar sind, so dass der Server (404) funktionsfähig ist, um das Verfahren nach einem der Ansprüche 1 bis 9 durchzuführen.

14. Client-Einrichtung (110; 502; 406), die ein Abrufen einer digitalen Repräsentation von Persönlichkeitsdaten eines Benutzers (402) von einem Server (404) ermöglicht, wobei die Client-Einrichtung (110; 502; 406) zumindest einen Prozessor (112) und zumindest einen Speicher (114) umfasst, wobei der zumindest eine Speicher (114) Instruktionen enthält, die von dem zumindest einen Prozessor (112) ausführbar sind, so dass die Client-Einrichtung (110; 502; 406) funktionsfähig ist, um das Verfahren nach Anspruch 10 durchzuführen.

15. System umfassend einen Server (100; 404) nach Anspruch 13 und zumindest eine Client-Einrichtung (110; 502; 406) nach Anspruch 14.

## Revendications

1. Procédé comprenant une récupération d'une représentation numérique de données de personnalité d'un utilisateur (402) par un dispositif client (502 ; 406) à partir d'un serveur (404), la représentation numérique des données de personnalité étant traitée au niveau du dispositif client (406) pour fournir à l'utilisateur (402) un service adapté à l'utilisateur, le procédé étant mis en œuvre par le serveur (404) et comprenant :
le stockage (S202) d'un réseau neuronal (602) entraîné pour calculer des données de personnalité d'un utilisateur (402) sur la base d'une entrée obtenue de l'utilisateur (402) ;
la réception (S204), en provenance du dispositif client (502 ; 406), d'une demande de représentation numérique de données de personnalité pour un utilisateur (402) ; et
l'envoi (S206), au dispositif client (502 ; 406), de la représentation numérique demandée des données de personnalité de l'utilisateur (402),
dans lequel la représentation numérique des données de personnalité de l'utilisateur (402) est traitée au niveau du dispositif client (502 ; 406) pour adapter une configuration d'au moins un dispositif (406) fournissant un service à l'utilisateur (402), dans lequel l'adaptation de la configuration dudit au moins un dispositif (406) comprend un élément parmi :
- l'adaptation d'une configuration de conduite d'un véhicule, dans laquelle ledit au moins un dispositif comprend un véhicule (406) et dans laquelle la représentation numérique des données de personnalité de l'utilisateur (402) est traitée au niveau du dispositif client (502 ; 406) pour adapter une configuration de conduite du véhicule (406) à une personnalité de l'utilisateur (402),
- l'adaptation d'une condition environnementale dans un habitacle d'un moyen de transport (406), dans laquelle ledit au moins un dispositif comprend le moyen de transport (406) et dans laquelle la représentation numérique des données de personnalité de l'utilisateur (402) est traitée au niveau du dispositif client (502 ; 406) pour adapter une condition environnementale dans l'habitacle du moyen de transport (406) à une personnalité de l'utilisateur (402),
- l'adaptation d'un paramètre spécifique à l'utilisateur concernant un habitacle d'un moyen de transport (406), dans laquelle ledit au moins un dispositif comprend le moyen de transport (406) et dans laquelle la représentation numérique des données de personnalité de l'utilisateur (402) est traitée au niveau du dispositif client (502 ; 406) pour adapter un paramètre spécifique à l'utilisateur concernant l'habitacle du moyen de transport (406) à une personnalité de l'utilisateur (402),
dans lequel l'adaptation de la configuration dudit au moins un dispositif (406) à la personnalité de l'utilisateur (402) est mise en œuvre au moyen de correspondances associant des caractéristiques de la personnalité de l'utilisateur (402), telles qu'indiquées par la représentation numérique des données de personnalité de l'utilisateur (402), à des configurations dudit au moins un dispositif (406),
dans lequel les données de personnalité de l'utilisateur (402) sont calculées à l'aide du réseau neuronal (602) sur la base d'une entrée obtenue de l'utilisateur (402), dans lequel le procédé comprend la réception d'un retour d'information caractérisant l'utilisateur (402), le retour d'information étant indicatif de la personnalité de l'utilisateur (402), et les données de personnalité de l'utilisateur (402) étant indicatives de caractéristiques psychologiques de l'utilisateur (402) et/ou de préférences de l'utilisateur (402) ;
la mise à jour du réseau neuronal (602) sur la base du retour d'information, la mise à jour du réseau neuronal comprenant l'entraînement du réseau neuronal sur la base du retour d'information ; et
l'envoi, au dispositif client (502 ; 406), d'une représentation numérique de secondes données de personnalité de l'utilisateur (402), les secondes données de personnalité de l'utilisateur (402) étant calculées à l'aide du réseau neuronal mis à jour (602),
dans lequel la représentation numérique des secondes données de personnalité de l'utilisateur (402) est traitée au niveau du dispositif client (502 ; 406) pour affiner la configuration dudit au moins un dispositif (406) fournissant à l'utilisateur (402) le service adapté à l'utilisateur.

2. Procédé selon la revendication 1, dans lequel ledit au moins un dispositif (406) comprend le dispositif client (406).

3. Procédé selon la revendication 1 ou 2, dans lequel le retour d'information est recueilli au niveau du dispositif client (110).

4. Procédé selon l'une quelconque des revendications 1 à 3, dans lequel le retour d'information comprend des données comportementales reflétant le comportement de l'utilisateur (402) surveillé au niveau dudit au moins un dispositif (406) lors de l'utilisation du service fourni par ledit au moins un dispositif (406), et, facultativement :
dans lequel les données comportementales sont surveillées à l'aide de mesures effectuées par ledit au moins un dispositif (406) fournissant le service à l'utilisateur (402).

5. Procédé selon la revendication 4, dans lequel ledit au moins un dispositif (406) comprend un véhicule et dans lequel les données comportementales comprennent des données reflétant un comportement de conduite de l'utilisateur (402).

6. Procédé selon l'une quelconque des revendications 1 à 5, dans lequel l'entrée obtenue de l'utilisateur correspond à des scores numériques reflétant des réponses à des questions concernant la personnalité de l'utilisateur (402), et, facultativement :
dans lequel chaque score numérique est utilisé comme entrée pour un nœud d'entrée distinct du réseau neuronal (602) lors du calcul des données de personnalité de l'utilisateur (402) à l'aide du réseau neuronal (602), et/ou
dans lequel les questions concernant la personnalité de l'utilisateur (402) correspondent à des questions issues d'au moins un des éléments suivants :
une banque internationale d'items de personnalité, IPIP,
un ensemble HEXACO-60,
un ensemble « Big-Five-Inventory-10 », BFI-10,
des questions sur des caractéristiques psychologiques de l'utilisateur (402), et
des questions sur les préférences de l'utilisateur (402).

7. Procédé selon la revendication 6, dans lequel les questions correspondent à des questions sélectionnées parmi un ensemble de questions représentatives d'un résultat pouvant être obtenu de manière optimale issu du calcul de données de personnalité d'un utilisateur (402), dans lequel les questions sélectionnées correspondent à des questions de l'ensemble de questions qui sont déterminées comme ayant le plus d'influence par rapport au résultat pouvant être obtenu de manière optimale, et, facultativement :
dans lequel le nombre de questions sélectionnées est inférieur à 10 % du nombre de questions comprises dans l'ensemble de questions.

8. Procédé selon la revendication 7, dans lequel les questions sont sélectionnées parmi l'ensemble de questions sur la base de la corrélation des résultats pouvant être obtenus par chaque question individuelle de l'ensemble de questions avec le résultat pouvant être obtenu de manière optimale et de la sélection des questions parmi l'ensemble de questions qui présentent la corrélation la plus élevée avec le résultat pouvant être obtenu de manière optimale, ou
dans lequel les questions sont sélectionnées de manière itérative parmi l'ensemble de questions, dans lequel, à chaque itération, une question suivante est sélectionnée en fonction d'une réponse de l'utilisateur à une question précédente, dans lequel, à chaque itération, la question suivante est sélectionnée comme étant une question parmi l'ensemble de questions qui est déterminée comme ayant le plus d'influence sur un résultat pouvant être obtenu pour le calcul de données de personnalité de l'utilisateur, et, facultativement :
dans lequel le réseau neuronal (602) comprend une pluralité de nœuds de sortie représentatifs d'une courbe de probabilité (604) d'un résultat des données de personnalité de l'utilisateur (402), dans lequel la détermination de la question ayant le plus d'influence parmi l'ensemble de questions en tant que question suivante de l'itération respective comprend la détermination, pour chaque nœud d'entrée du réseau neuronal (602), d'un degré selon lequel une modification du score numérique fourni en entrée au nœud d'entrée respectif du réseau neuronal (602) modifie la courbe de probabilité (604).

9. Procédé selon l'une quelconque des revendications 1 à 8, dans lequel :
(a) les données de personnalité de l'utilisateur (402) sont calculées avant la réception de la demande du dispositif client (502 ; 406) et dans lequel la demande comprend un code d'accès préalablement fourni par le serveur (404) à l'utilisateur (402) lors du calcul des données de personnalité de l'utilisateur (402), le code d'accès permettant à l'utilisateur (402) d'accéder à la représentation numérique des données de personnalité de l'utilisateur (402) depuis différents dispositifs clients (502 ; 406) ; et/ou
(b) lorsque ledit au moins un dispositif (406) comprend un moyen de transport, la fourniture à l'utilisateur (402) du service adapté à l'utilisateur est en outre effectuée en tenant compte de données de capteur indicatives d'un niveau d'attention de l'utilisateur (402) obtenues dans un habitacle du moyen de transport (406) ; et/ou
(c) la fourniture à l'utilisateur (402) du service adapté à l'utilisateur est en outre effectuée en tenant compte de données de balayage corporel indicatives de caractéristiques de l'utilisateur (402) pouvant être dérivées par balayage d'au moins une partie du corps de l'utilisateur (402).

10. Procédé comprenant une récupération d'une représentation numérique de données de personnalité d'un utilisateur (402) par un dispositif client (502 ; 406) à partir d'un serveur (404), le procédé étant mis en œuvre par le dispositif client (502 ; 406) et comprenant :
l'envoi (S302), au serveur (404), d'une demande de représentation numérique de données de personnalité pour un utilisateur (402) ;
la réception (S304), en provenance du serveur (404), de la représentation numérique demandée des données de personnalité de l'utilisateur (402), les données de personnalité de l'utilisateur (402) étant calculées, sur la base d'une entrée obtenue de l'utilisateur (402), à l'aide d'un réseau neuronal (602) entraîné pour calculer des données de personnalité pour un utilisateur (402) sur la base d'une entrée obtenue de l'utilisateur (402) ; et
le traitement (S306) de la représentation numérique des données de personnalité pour fournir à l'utilisateur (402) un service adapté à l'utilisateur,
dans lequel la représentation numérique des données de personnalité de l'utilisateur (402) est traitée au niveau du dispositif client (502 ; 406) pour adapter une configuration d'au moins un dispositif (406) fournissant un service à l'utilisateur (402), dans lequel l'adaptation de la configuration dudit au moins un dispositif (406) comprend un élément parmi :
- l'adaptation d'une configuration de conduite d'un véhicule, dans laquelle ledit au moins un dispositif comprend un véhicule (406) et dans laquelle la représentation numérique des données de personnalité de l'utilisateur (402) est traitée au niveau du dispositif client (502 ; 406) pour adapter une configuration de conduite du véhicule (406) à une personnalité de l'utilisateur (402),
- l'adaptation d'une condition environnementale dans un habitacle d'un moyen de transport (406), dans laquelle ledit au moins un dispositif comprend le moyen de transport (406) et dans laquelle la représentation numérique des données de personnalité de l'utilisateur (402) est traitée au niveau du dispositif client (502 ; 406) pour adapter une condition environnementale dans l'habitacle du moyen de transport (406) à une personnalité de l'utilisateur (402),
- l'adaptation d'un paramètre spécifique à l'utilisateur concernant un habitacle d'un moyen de transport (406), dans laquelle ledit au moins un dispositif comprend le moyen de transport (406) et dans laquelle la représentation numérique des données de personnalité de l'utilisateur (402) est traitée au niveau du dispositif client (502 ; 406) pour adapter un paramètre spécifique à l'utilisateur concernant l'habitacle du moyen de transport (406) à une personnalité de l'utilisateur (402),
dans lequel l'adaptation de la configuration dudit au moins un dispositif (406) à la personnalité de l'utilisateur (402) est mise en œuvre au moyen de correspondances associant des caractéristiques de la personnalité de l'utilisateur (402), telles qu'indiquées par la représentation numérique des données de personnalité de l'utilisateur (402), à des configurations dudit au moins un dispositif (406),
dans lequel le procédé comprend en outre :
l'envoi, au serveur (404), d'un retour d'information caractérisant l'utilisateur (402), le retour d'information étant indicatif de la personnalité de l'utilisateur (402), et les données de personnalité de l'utilisateur (402) étant indicatives de caractéristiques psychologiques de l'utilisateur (402) et/ou de préférences de l'utilisateur (402) ; et
la réception, en provenance du serveur (404), d'une représentation numérique de secondes données de personnalité de l'utilisateur (402), dans laquelle les secondes données de personnalité de l'utilisateur (402) sont calculées à l'aide du réseau neuronal (602) mis à jour sur la base du retour d'information, dans laquelle la mise à jour du réseau neuronal comprend l'entraînement du réseau neuronal sur la base du retour d'information,
dans lequel la représentation numérique des secondes données de personnalité de l'utilisateur (402) est traitée au niveau du dispositif client (502 ; 406) pour affiner la configuration dudit au moins un dispositif (406) fournissant à l'utilisateur (402) le service adapté à l'utilisateur.

11. Produit programme d'ordinateur comprenant des parties de code de programme pour mettre en œuvre le procédé selon l'une quelconque des revendications 1 à 10 lorsque le produit programme d'ordinateur est exécuté sur une ou plusieurs unités de calcul (102).

12. Support d'enregistrement lisible par ordinateur sur lequel est stocké le produit programme d'ordinateur selon la revendication 11.

13. Serveur (100 ; 404) permettant la récupération d'une représentation numérique de données de personnalité d'un utilisateur (402) par un dispositif client (502 ; 406) à partir du serveur (404), la représentation numérique des données de personnalité étant traitée au niveau du dispositif client (502 ; 406) pour fournir à l'utilisateur (402) un service adapté à l'utilisateur, le serveur (404) comprenant au moins un processeur (102) et au moins une mémoire (104), ladite au moins une mémoire (104) contenant des instructions exécutables par ledit au moins un processeur (102) de sorte que le serveur (404) est utilisable pour mettre en œuvre le procédé selon l'une quelconque des revendications 1 à 9.

14. Dispositif client (110 ; 502 ; 406) permettant la récupération d'une représentation numérique de données de personnalité d'un utilisateur (402) à partir d'un serveur (404), le dispositif client (110 ; 502 ; 406) comprenant au moins un processeur (112) et au moins une mémoire (114), ladite au moins une mémoire (114) contenant des instructions exécutables par ledit au moins un processeur (112) de sorte que le dispositif client (110 ; 502 ; 406) est utilisable pour mettre en œuvre le procédé selon la revendication 10.

15. Système comprenant un serveur (100 ; 404) selon la revendication 13 et au moins un dispositif client (110 ; 502 ; 406) selon la revendication 14.
